# EUROPEAN PATENT SPECIFICATION

(11) **EP 2 949 264 B1**
(45) Date of publication and mention of the grant of the patent: **15.02.2017**
(21) Application number: 15169190.4
(22) Date of filing: 26.05.2015
(51) Int. Cl.: A61B 3/00, A61B 3/10

(54) **OPHTHALMIC APPARATUS**
OPHTHALMISCHE VORRICHTUNG
APPAREIL OPHTALMIQUE

(30) Priority: 30.05.2014 JP 2014113071
(43) Date of publication of application: 02.12.2015
(73) Proprietor: TOMEY CORPORATION, Nagoya-shi, Aichi 451-0051 (JP)
(72) Inventor: KATO, Chihiro, Nagoya-shi, Aichi 451-0051 (JP); NOZAWA, Yuji, Nagoya-shi, Aichi 451-0051 (JP)
(74) Representative: Kramer Barske Schmidtchen Patentanwälte PartG mbB

(56) References cited:
- JP-A- 2008 175 698
- US-A1- 2007 035 743
- US-A1- 2012 316 434
- US-A1- 2013 128 274
- US-A1- 2013 229 627

## Description

### CROSS-REFERENCE TO RELATED APPLICATION

This application claims priority to Japanese Patent Application No. 2014-113071, filed on May 30, 2014, the contents of which are hereby incorporated by reference into the present application.

### TECHNICAL FIELD

The present teachings relate to an ophthalmic apparatus for examining an eye.

### DESCRIPTION OF RELATED ART

Japanese Patent Application Publication No. 2007-37984 discloses an ophthalmic apparatus for examining an interior (e.g., crystalline lens, retina) of an eye. This ophthalmic apparatus is equipped with an optical measurement system for radiating light from a light source to the interior of the eye and guiding reflected light, and an optical reference system for radiating light from the light source to a reference surface and guiding the reflected light. Inside of the eye is examined using interference light produced by both the reflected light guided by the optical measurement system and the reflected light guided by the optical reference system.

Document US 2012/0316434 shows a device according to the preamble of claim 1.

### BRIEF SUMMARY

Methods of examining an interior of an eye to be examined by using interference light includes a time domain method and a Fourier domain method. As compared with the time domain method, the Fourier domain method has an advantage in that it can obtain data at high speed. The Fourier domain method may use a light source of a wavelength sweeping type, but use of the light source of the wavelength sweeping type changes wavelength sweeping properties of light outputted from the light source, such as by the change caused by aging. As a result, a problem that measurement sensitivity of the ophthalmic apparatus or an image quality of captured image decreases occurs.

It is an object of the present teachings to provide an ophthalmic apparatus capable of maintaining the measurement sensitivity of the ophthalmic apparatus or the image quality of captured image when the wavelength sweeping properties of the light outputted from the light source of the wavelength sweeping type change, such as by the change caused by aging.

An ophthalmic apparatus disclosed in the present specification examines an eye by using interference light obtained from the reflected light reflected from the eye. This ophthalmic apparatus includes a light source device of wavelength sweeping type, an optical reference system, a calibration member, a light receiving element, and a control unit. The light source device includes a light source, a moving part, and a driving part configured to periodically drive the moving part, by the light from the light source being outputted via the moving part that is driven periodically. Due to this periodical drive of the moving part a wavelength of the light outputted from the light source device is swept periodically. The optical reference system is configured to guide light from the light source device as reference light. The calibration member is configured to have a predetermined setting length as an optical path length from the light source device, and include a reflection surface for reflecting light from the light source device. The light receiving element receives interference light for calibration produced by both of reflected light reflected at the reflection surface of the calibration member and the reference light guided by the optical reference system. The light receiving element outputs an interference signal for calibration when the interference light for calibration is received. The control unit obtains, according to a driving cycle of the moving part, a reference point spread function signal by obtaining the interference signal for calibration output from the light receiving element during a given obtainment period in the driving cycle. When a peak shape of the reference point spread function signal deviates from a predetermined shape range, the control unit adjusts a timing to start the obtainment period in the driving cycle of the moving part so that the peak shape of the reference point spread function signal comes to be within predetermined shape range.

The ophthalmic apparatus radiates light from the light source device of the wavelength sweeping type to the reflection surface of the calibration member, and forms the interference light for calibration by combining the reflected light and the reference light. The reference point spread function signal is obtained by obtaining the interference signal for calibration, which is obtained from the interference light for calibration, within the predetermined obtainment period of the driving cycle of the light source device. Herein, if the wavelength sweeping properties of the light source device do not change, the peak shape of the reference point spread function signal does not change. That is, when the measurement sensitivity or the image quality of captured image is maintained high without changing the wavelength sweeping properties of the light source device, the peak shape becomes sharp, wherein the height of the peak shape becomes high and the width of the peak shape becomes narrow. Therefore, when the wavelength sweeping properties of the light source change with lapse of time and the measurement sensitivity or the image quality of captured image decreases, the peak shape of the reference point spread function signal changes. That is, the peak shape becomes blunt, wherein the height of the peak shape becomes low, and the width of the peak shape becomes wide. Accordingly, the ophthalmic apparatus determines whether or not the change of the wavelength sweeping properties of the light device is within its tolerable range based on whether or not the peak shape of the reference point spread function signal is within the predetermined shape range. The timing to start the signal obtainment period in the driving cycle of the moving part is adjusted, so that the peak shape of the reference point spread function signal is within the predetermined shape range. According to the aforementioned configurations, the peak shape of the reference point spread function signal comes to be within the predetermined shape range, and the measurement sensitivity or the image quality of captured image can be maintained within the tolerable range.

### BRIEF DESCRIPTION OF DRAWINGS

FIG. 1 is a diagram schematically showing a configuration of an optical system of an ophthalmic apparatus according to an embodiment;
FIG. 2 is a block diagram of a control system of the ophthalmic apparatus according to the embodiment;
FIG. 3 is a diagram for illustrating a positional relationship of zero point, a reference mirror, and an eye to be examined;
FIG. 4 is a diagram for illustrating a procedure of processing an interference signal waveform obtained when scanning an optical path length of an optical measurement system within a predetermined range of the optical path length;
FIG. 5 is a flow chart showing an example of a processing procedure of the ophthalmic apparatus according to the embodiment;
FIG. 6 is a diagram schematically showing a reference point spread function signal obtained from an interference signal for calibration, where (a) shows the reference point spread function signal in an initial state, (b) shows the reference point spread function signal of a light source device that had experienced aging; and
FIG. 7 is a diagram collectively showing a change of a wavelength of light outputted from light source device, a driving voltage signal for driving a MEMS mirror of the light source device, and a trigger signal.

### DETAILED DESCRIPTION OF EMBODIMENTS

In one aspect of the present teachings, a driving part may drive a moving part according to a trigger signal that is periodically outputted. The control unit may obtain a reference point spread function signal by starting an obtainment period when a set time has elapsed since when the trigger signal had been outputted. In this case, when the peak shape of the reference point spread function signal deviates from the predetermined shape range, a control unit may adjust the set time from the output of the trigger signal to the start of the obtainment period. According to such a configuration, the moving part is driven using the trigger signal, and the timing to start the obtainment period is adjusted, so the peak shape of the reference point spread function signal can properly be adjusted by a simple method.

In one aspect of the present teachings, when a height of the peak shape of the reference point spread function signal becomes lower than a predetermined set height, the control unit may adjust the timing to start the obtainment period in the driving cycle of the moving part, so that the height of the peak shape of the reference point spread function signal becomes higher than the predetermined set height. According to such a configuration, a change in the peak shape can be detected by using the height of the peak shape of the reference point spread function signal as a reference.

In one aspect of the present teachings, when a width of the peak shape of the reference point spread function signal becomes wider than a predetermined set width, the control unit may adjust the timing to start the obtainment period in the driving cycle of the moving part, so that the width of the peak shape of the reference point spread function signal becomes narrower than the predetermined set width. According to such a configuration, the change in the peak shape can be detected by using the width of the peak shape of the reference point spread function signal as the reference.

In one aspect of the present teachings, when a peak position of the reference point spread function signal deviates from a predetermined peak position range, the control unit may adjust the wavelength sweep width of the light source device by controlling the driving part, so that the peak position of the reference point spread function signal comes to be within the predetermined peak position range. The change in the peak position of the reference point spread function signal occurs by the change in the wavelength sweep width of the light outputted from the light source device. Accordingly, measurement sensitivity of an ophthalmic apparatus or image quality of a captured image can be increased by adjusting the wavelength sweep width of the light source device.

In one aspect of the present teachings, the driving part may drive the moving part by outputting a pulsed drive signal. In this case, the control unit may adjust the wavelength sweep width of the light source device by adjusting the pulse width of the drive signal outputted from the driving part. Alternatively, the control unit may adjust the wavelength sweep width of the light source device by adjusting the cycle of the drive signal outputted from the driving part. According to such a configuration, the wavelength sweep width of the light source device can be adjusted easily by adjusting the pulse width of the pulsed drive signal (in the height direction and/or lateral direction) for driving the moving part or the output cycle of the drive signal.

Note that, in another aspect of the present teachings, an optical measurement system may be configured to radiate light from the light source to an interior of an eye to be examined, and guide reflected light from the eye. In this case, a light receiving element may receive interference light for measurement produced by combining both the reflected light guided by the optical measurement system and the reference light guided by the optical reference system. The control unit may determine a position of a measuring portion inside the eye by Fourier-analyzing the interference light for measurement received by the light receiving element.

### (Embodiment 1)

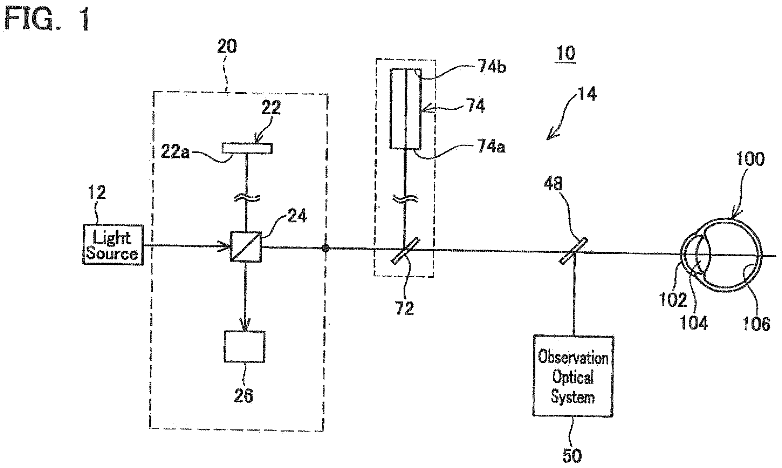

As shown in FIG. 1, an ophthalmic apparatus according to a representative embodiment of the present teachings includes a measuring unit 10 for examining an eye 100 to be examined (hereinbelow may be referred to simply as "the eye 100"). The measuring unit 10 includes an optical interference system 14 for causing interference between reflected light reflected from the eye 100 and reference light, an optical observation system 50 for observing an anterior eye part of the eye 100, and an optical alignment system (not shown) for aligning the measuring unit 10 with respect to the eye 100 such that the measuring unit 10 exhibits a predetermined positional relationship with the eye 100. An optical alignment system used in a known ophthalmic apparatus may be utilized as the optical alignment system, hence the detailed description thereof will be omitted.

The optical interference system 14 includes a light source device 12, an optical measurement system (24, 72, 48) for radiating light from the light source device 12 to the interior of the eye 100 and guiding reflected light from the eye 100, an optical reference system (24, 22) for radiating light from the light source device 12 on a reference surface 22a and guiding reflected light therefrom, an optical calibration system (24, 72, 74) for radiating light from the light source device 12 on reflection surfaces (74a, 74b) and guiding reflected light therefrom, and a light receiving element 26 for receiving interference light for measurement and interference light for calibration. The interference light for measurement is produced by combining the reflected light guided by the optical measurement system and the reflected light guided by the optical reference system. The interference light for calibration is produced combining both by the reflected light guided by the optical calibration system and the reflected light guided by the optical reference system.

The light source device 12 is a light source of a wavelength sweeping type, and a wavelength of light to be radiated is to be changed at a predetermined cycle. That is, in the ophthalmic apparatus according to the embodiment, the light radiated from the light source device 12 is radiated to the eye 100 while changing its wavelength (i.e., scanning). Further, an intensity distribution of the light reflected from each portion of the eye 100 in a depth direction is obtained by Fourier-analyzing a signal obtained from interference light produced by the reflected light from the eye 100 and reference light. When the light intensity distribution of the eye 100 in the depth direction is obtained, it becomes possible to specify a position of each portion (that is, a crystalline lens 104 and a retina 106) inside the eye 100 as described below. Note that, the wavelength sweeping properties of light radiated from the light source device 12 is changed as a result of aging of the light source device 12. Accordingly, a result of the Fourier analysis of the signal obtained from interference light is also changed by the aging the ophthalmic apparatus.

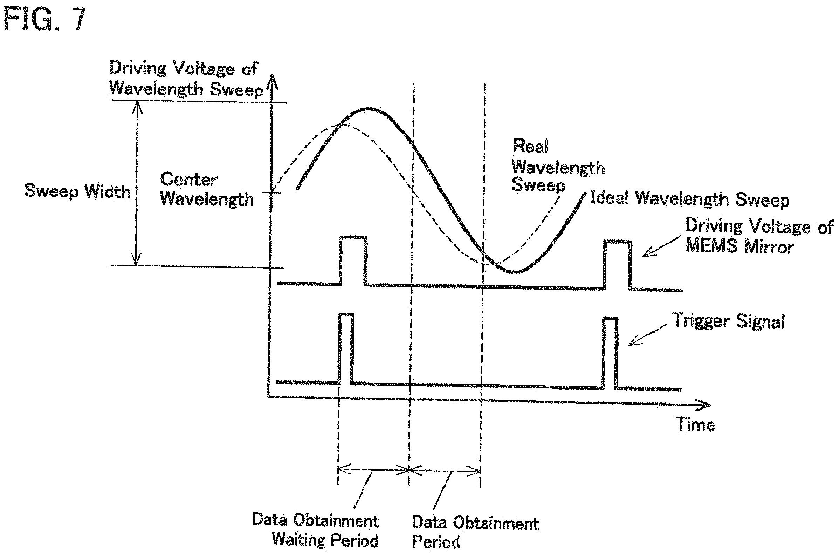

For example, the light source device 12 includes a laser light source (coherence light source), a wavelength sweeping element (MEMS mirror), a wavelength select element (diffraction grating), and the driving circuit. That is, the light from the laser source is guided to the wavelength select element (diffraction grating) via the wavelength sweeping element (MEMS mirror). Among the light guided to the wavelength select element (diffraction grating), only the light in a predetermined wavelength is outputted to the outside via the wavelength select element (diffraction grating). By the driving circuit driving the wavelength sweeping element (MEMS mirror) periodically, the wavelength of the light that can pass through the wavelength select element (diffraction grating) changes periodically. Due to the above, the wavelength of the light outputted from the light source device 12 changes periodically. Specifically, as shown in FIG. 7, the driving circuit outputs the pulsed drive signal to the wavelength sweeping element (MEMS mirror) according to the trigger signal periodically outputted from a processor 64. Due to this, the wavelength sweeping element (MEMS mirror) is driven, and the light from the laser light source is introduced to the wavelength select element (diffraction grating) in different incident angles. As a result, the wavelength of the light outputted from the light source device 12 changes periodically. Note that, in addition to the MEMS mirror, a polygon mirror or a movable mirror may be used as the wavelength sweeping element.

The optical measurement system includes a beam splitter 24, a beam splitter 72, and a hot mirror 48. The light radiated from the light source device 12 is radiated to the eye 100 via the beam splitter 24, the beam splitter 72, and the hot mirror 48. The reflected light from the eye 100 is guided to the light receiving element 26 via the hot mirror 48, the beam splitter 72 and the beam splitter 24.

The optical reference system includes the beam splitter 24 and the reference mirror 22. Some of the light radiated from the light source device 12 is reflected by the beam splitter 24, radiated on a reference surface 22a of the reference mirror 22, and reflected by the reference surface 22a of the reference mirror 22. The light reflected by the reference mirror 22 is guided to the light receiving element 26 via the beam splitter 24. The reference mirror 22, the beam splitter 24, and the light receiving element 26 are disposed in an interferometer 20, and their positions are fixed. Accordingly, in the ophthalmic apparatus according to the embodiment, a reference optical path length of the optical reference system is constant and is not changed.

The calibration system includes the beam splitter 24, the beam splitter 72, and an optical member 74. The light radiated from the light source device 12 is radiated to the optical member 74 via the beam splitter 24 and the beam splitter 72. The optical member 74 has a first reflection surface 74a provided on an end thereof and a second reflection surface 74b provided on the other end thereof. Accordingly, some of the light radiated to the optical member 74 is reflected by the first reflection surface 74a and remaining light is introduced in the optical member 74. Some of the light introduced in the optical member 74 is reflected by the second reflection surface 74b and remaining light is radiated outside form the optical member 74. The light reflected by the first reflection surface 74a and the light reflected by the second reflection surface 74b is guided to the light receiving element 26 via the beam splitter 72 and the beam splitter 24.

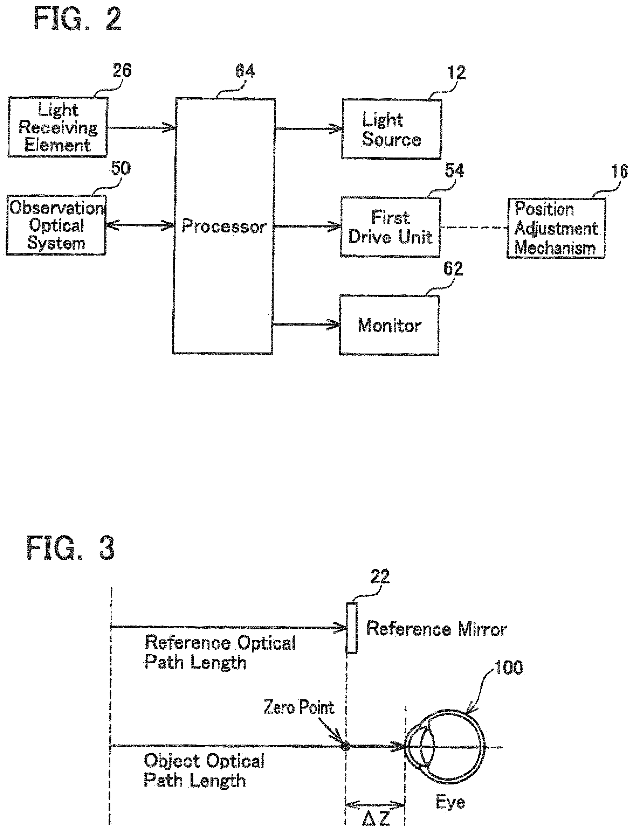

The optical calibration system according to the embodiment has a first optical path section (i.e., light source device 12 → first reflection surface 74a → light receiving element 26) set to have a first optical path length from zero point, and a second optical path section (i.e., light source device 12 → second reflection surface 74b → light receiving element 26) set to have a second optical path length from the zero point. Specifically, a position of the optical member 74 is set based on the zero point, and an optical path length from the zero point to the first reflection surface 74a and an optical path length from the zero point to the second reflection surface 74b are set longer than a distance from the zero point to the retina of the eye 100. Herein, the zero point denotes a point where an optical path length of the optical reference system (reference optical path length) and an optical path length of an optical object system (object optical path length) match (see FIG. 3). As is apparent from the above description, a difference of the optical path lengths of the fist optical path section and the second optical path section is determined by a length from the first reflection surface 74a, which is an end of the optical member 74, to the second reflection surface 74b, which is the other end of the optical member 74. That is, the difference of the optical path lengths of the first optical path section and the second optical path section is not influenced by a positional relationship between the optical member 74 and other members, and is determined only by the optical member 74. Accordingly, the difference of the optical path lengths of the first optical path section and the second optical path section can be managed with high accuracy by increasing shape accuracy of the optical member 74. Note that, only the light reflected by the first reflection surface 74a of the optical member 74 is used in order to adjust of the wavelength sweeping properties of the light source device 12 as described below.

Note that, for example, an optical glass may be used for the optical member 74. By radiating light from the light source device 12 to the optical glass, an end of the optical glass (incident surface) can be functioned as the first reflection surface and the other end of the optical glass (emission surface) can be functioned as the second reflection surface. Another example of the optical member 74 includes, for example, an optical plastic.

The light receiving element 26 detects interference light for measurement and interference light for calibration. The interference light for measurement is produced by combining the light guided by the optical reference system and the light guided by the optical measurement system, and the interference light for calibration is produced by combining both the light guided by the optical reference system and the light guided by the optical calibration system. As is apparent from the above description, the interference light for calibration includes first interference light for calibration and second interference light for calibration. The first interference light for calibration is produced by both the light reflected by the first reflection surface 74a (i.e., light guided by the first optical path section) and light guided by the optical reference system. The second interference light for calibration is produced by both the light reflected by the second reflection surface 74b (i.e., light guided by the second optical path section) and the light guided by the optical reference system. Accordingly, the light receiving element 26 detects the interference light for measurement, the first interference light for calibration, and the second interference light for calibration. When the light receiving element 26 receives the interference light for measurement, the first interference light for calibration, and the second interference light for calibration, it outputs interference signals corresponding thereto. That is, the light receiving element 26 outputs the signal by the interference light for measurement (the interference signal for measurement), the signal by the first interference light for calibration (the first interference signal for calibration), and the signal by the second interference light for calibration (the second interference signal for calibration). These signals are inputted to the processor 64. For example, a photodiode may be used for the light receiving element 26. Note that, as described above, only the first interference signal for calibration is used in order to the adjust of the wavelength sweeping properties of the light source device 12. Accordingly, the adjustment of the wavelength sweeping properties of the light source device 12 can be executed despite an examination of the eye 100 is to be performed or not.

The optical observation system 50 radiates observation light to the eye 100 via the hot mirror 48 and captures an image from the reflected light reflected from the eye 100 (that is, reflected light being the reflection of the radiated observation light). Herein, the hot mirror 48 reflects the light from the light source device 12 while transmits the light from the light source of the optical observation system 50. Accordingly, in the ophthalmic apparatus according to the embodiment, measurement by the optical interference system and the observation of the anterior eye part by the optical observation system 50 can be executed at a same time. Note that, since an optical observation system used in a known ophthalmic apparatus may be utilized as the optical observation system 50, detailed description of a configuration thereof will be omitted.

Note that, the ophthalmic apparatus according to the embodiment includes a position adjusting mechanism 16 for adjusting a position of the measuring unit 10 with respect to the eye 100 (see FIG. 2) and a first driving device 54 for driving the position adjusting mechanism 16 (see FIG. 2). The position of the measuring unit 10 with respect to the eye 100 is adjusted by actuating the first driving device 54.

Next, a configuration of a control system of the ophthalmic apparatus according to the embodiment will be described. As shown in FIG. 2, the ophthalmic apparatus is controlled by a processor 64. The processor 64 includes a microcomputer (microprocessor) including a CPU, a ROM, a RAM, etc., and a gate array for high-speed computing. The light source device 12, the first driving device 54, a monitor 62, and the optical observation system 50 are connected to the processor 64. The processor 64 controls on/off of the light source device 12, and controls wavelength sweep of light outputted from the light source device 12. Specifically, the processor 64 controls on/off of a laser source of the light source device 12, and outputs the trigger signal to the driving circuit of the light source device 12. Due to this, the wavelength of the light output from the light source device 12 is periodically swept. The processor 64 also drives the position adjusting mechanism 16 by controlling the first driving device 54, and controls the optical observation system 50 to display the image of the anterior eye part captured by the optical observation system 50 on the monitor 62.

The light receiving element 26 is also connected to the processor 64, and an interference signal depending on the intensity of the interference light (i.e., interference light for measurement, first interference light for calibration, second interference light for calibration) detected by the light receiving element 26 is inputted to the processor 64. The processor 64 obtains the input interference signal from the light receiving element 26 at a predetermined timing. Specifically, as shown in FIG. 7, obtainment of the interference signal is not performed for a predetermined period from when the trigger signal was sent to the light source device 12 (data obtainment waiting period), and then the interference signal is obtained in a predetermined period after the predetermined data obtainment waiting period has passed (data obtainment period). Due to this, the data is obtained in a period in which the wavelength of light outputted from the light source device 12 changes linearly. Accordingly, the eye 100 can be examined with high accuracy. Note that, a relationship between the trigger signal and the wavelength sweep of light outputted from the light source device 12 changes due to aging of the light source device 12 (e.g., wavelength sweep of the solid line in FIG. 7 → wavelength sweep of the dot line in FIG. 7). As a result, the data obtainment period for obtaining the interference signal deviates, and the measurement sensitivity or the image quality of the captured image deteriorates. Therefore, in the ophthalmic apparatus according to the embodiment, the first interference signal for calibration is used in order to adjust the wavelength sweeping properties of the light source device 12. Details of process to adjust the wavelength sweeping properties of the light source device 12 by the processor 64 will be described later.

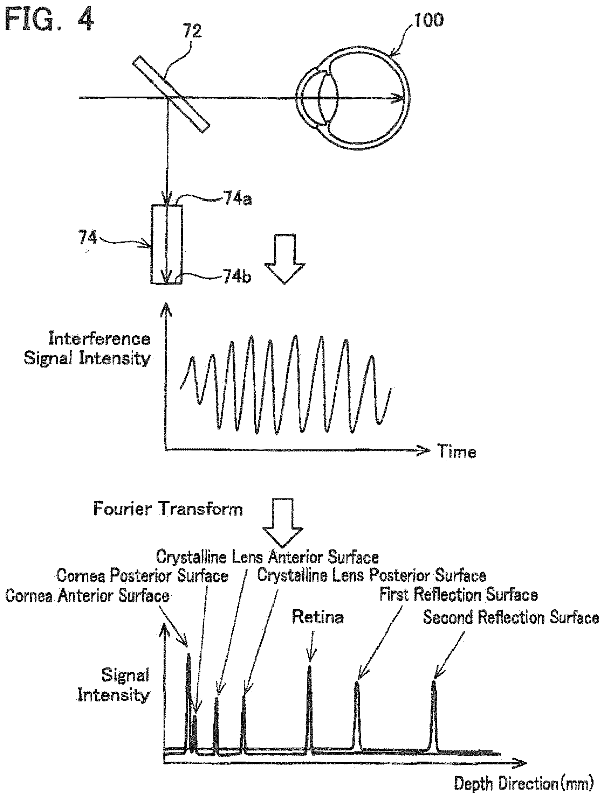

The processor 64 specifies positions of portions of the eye 100 (e.g., front and rear surfaces of the cornea 102, front and rear surfaces of the crystalline lens 104, a surface of the retina 106) and the reflection surfaces 74a and 74b of the optical member 74 by Fourier transforming the interference signal from the light receiving element 26 obtained as described above, and calculates an ocular axial length of the eye 100 using the specified positions. That is, the interference signal outputted from the light receiving element 26 becomes a signal of which signal intensity is changed depending on time as shown in FIG. 4, and the signal includes a signal depending on an interference wave produced by both the reference light and reflected light that is reflected from the respective portions of the eye 100 (i.e., the front surface and rear surface of the cornea 102, the front surface and rear surface of the crystalline lens 104, the surface of the retina 106) and the first and second reflection surfaces 74a and 74b. Therefore, the processor 64 performs Fourier transform of the signal inputted from the light receiving element 26 to thereby divide, from the signal, an interference signal component depending on the reflected light reflected from each portion of the eye 100 (i.e., the front surface and rear surface of the cornea 102, the front surface and rear surface of the crystalline lens 104, the surface of the retina 106) and the first and second reflection surfaces 74a and 74b (see a bottom graph in FIG. 4). Herewith, the processor 64 can specify the position of each portion of the eye 100 and the positions of the first and second reflection surfaces 74a and 74b. The ocular axial length of the eye 100 can be calculated by specifying the position of each portion of the eye 100.

Next, a procedure of the adjustment of the wavelength sweeping properties of the light source device 12 by using the ophthalmic apparatus according to the embodiment will be described. As described above, the adjustment of the wavelength sweeping properties of the light source device 12 can be executed despite the eye 100 is examined or not. Accordingly, the adjustment of the wavelength sweeping properties of the light source device 12 may be executed periodically each time a predetermined period elapses (e.g., every month), or may be executed at any timing, including upon a startup of the ophthalmic apparatus (energization start time) or upon shifting to measurement or imaging mode, etc.

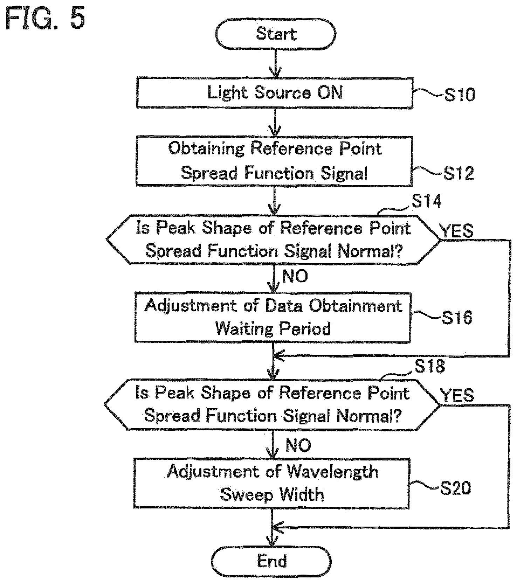

As illustrated in FIG. 5, first, the processor 64 starts driving the light source device 12, and starts outputting the light from the light source device 12 (S10). That is, the processor 64 turns ON the laser source of the light source device 12 and outputs the trigger signal to a driving circuit of the light source device 12. The driving circuit of the light source device 12 outputs the driving signal (pulse signal) to a wavelength sweeping element depending on the trigger signal. The wavelength of the light output from the light source device 12 is thereby swept.

Next, the processor 64 obtains the input interference signal from the light receiving element 26 at a predetermined timing, and obtains the reference point spread function signal by performing Fourier transformation on the obtained interference signal (S12). That is, the processor 64 waits to obtain the interference signal predetermined data during the obtainment waiting period that starts when the trigger signal was outputted to the light source device 12, and then obtains the interference signal predetermined data only during the obtainment period. Next, the processor 64 performs Fourier transformation on the interference signal obtained as described above during the obtainment period. Herein, the interference signal includes a signal corresponding to the interference wave produced by combining the reference light, and the reflected light reflected from the first reflection surface 74a and the second reflection surface 74b of the optical member 74. Accordingly, the processor 64 divides the interference signal into the interference signal component of the reflected light reflected from the first reflection surface 74a and the interference signal component of the reflected light reflected from the second reflection surface 74b by performing Fourier transformation on the obtained interference signal. As a result, the signal of the interference signal component of the reflected light reflected from the first reflection surface 74a, that is, the reference point spread function signal, is obtained.

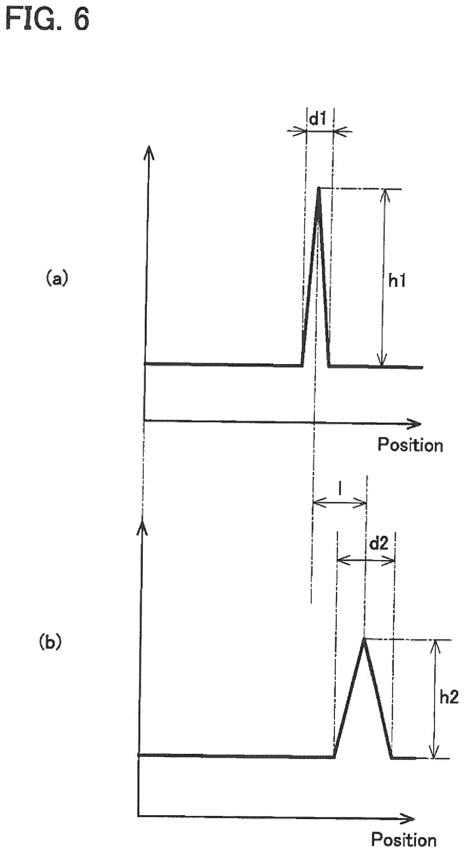

Next, the processor 64 determines whether or not a peak shape of the reference point spread function signal obtained in step S12 is out of a predetermined shape range (normal shape range) (S14). Specifically, as illustrated in FIG. 6, a height h and a width d of the peak shape of the reference point spread function signal is determined as to whether they are within a predetermined range. That is, when the aging of the light source device 12 has not occurred yet, as illustrated in FIG. 6(a), the height h1 of the peak shape of the reference point spread function signal is high, and the width d1 is narrow. Therefore, when the wavelength sweeping properties of the light source device 12 change by the occurrence of the aging of the light source device 12, as illustrated in FIG. 6(b), the height h2 of the peak shape of the reference point spread function signal becomes lower, and the width d2 becomes wider. Accordingly, in step S14, the processor 64 determines that the peak shape of the reference point spread function signal is out of the predetermined shape range when the height h of the peak shape of the reference point spread function signal obtained in step S12 becomes lower than a predetermined height and the width d of the peak shape becomes wider than a predetermined width. Therefore, the processor 64 determines that the peak shape of the reference point spread function signal is not out of the predetermined shape range when the height h of the peak shape of the reference point spread function signal obtained in step S12 is higher than the predetermined height or the width d of the peak shape is narrower than the predetermined width. When the peak shape of the reference point spread function signal is determined as not having deviated from the predetermined shape range (YES in step S14), the process skips step S16, and proceeds to step S18.

When the peak shape of the reference point spread function signal is determined to be deviated from the predetermined shape range (NO in step S14), the processor 64 adjusts the data obtainment waiting period that is from the output of the trigger signal to the start of the data obtainment period (S16). That is, the present inventors have found, as a result of intensive study, that the cause of the deviation of the peak shape of the reference point spread function signal from the predetermined shape range was due to a shift in a phase of the data obtainment period relative to a cycle of the wavelength sweep of the light source device 12. Accordingly, the processor 64 adjusts the data obtainment waiting period so that the peak shape of the reference point spread function signal comes to be within the predetermined shape range. That is, the data obtainment waiting period is made longer when the peak shape approaches the normal range by lengthening the data obtainment waiting period, on the other hand, the data obtainment waiting period is made shorter when the peak shape approaches the normal range by shortening the data obtainment waiting period. Note that, when the peak shape of the reference point spread function signal deviates from the predetermined shape range, the image quality of the captured image obtained from the ophthalmic apparatus or the measurement sensitivity is deteriorated. In step S16, by adjusting the data obtainment waiting period such that the peak shape of the reference point spread function signal comes to be within predetermined shape range, the deterioration of the image quality of the captured image obtained from the ophthalmic apparatus or the measurement sensitivity is suppressed. Herewith, the eye 100 can be examined with high accuracy.

Note that, in the according to the embodiment, the peak shape is determined to deviate from a set range when both the height and width of the peak shape of the reference point spread function signal deviate from the set range, however the peak shape may alternatively be determined to deviate from the set range when at least one of the height and width of the peak shape deviates. By determining in this way, the determination on the normality of the peak shape is performed more strict, and adjustment that better suits the aging of the light source device 12 can be performed.

In step S18, the processor 64 determines whether the peak position of the reference point spread function signal obtained in step S12 is out of the predetermined position range (normal position range). That is, since the position of the first reflection surface 74a of the optical member 74 is fixed and does not change, if the wavelength sweeping properties of the light source device 12 do not change, the peak position of the reference point spread function signal (that is, equivalent to the position of the first reflection surfaces 74a) also becomes fixed. Therefore, if the wavelength sweeping properties of the light source device 12 change, the peak position of the reference point spread function signal (that is, equivalent to the position of the first reflection surface 74a) changes (e.g., the state shown in FIG. 6(a) → the state shown in FIG. 6(b)). Accordingly, in step S18, the processor 64 determines that the peak position of the reference point spread function signal is out of the predetermined position range (normal position range) when the difference 1 of the peak position of the reference point spread function signal obtained in step S12 (e.g., the peak position shown in FIG. 6(b)) and the position of the reference point spread function signal in the initial state (e.g., the peak position shown in FIG. 6(a)) becomes larger than a setting value. When the peak position of the reference point spread function signal is determined to have not deviated from the predetermined position range (YES in step S18), step S20 is skipped, and the process is finished.

When the peak position of the reference point spread function signal is determined to be deviated from the predetermined position range (NO in step S18), the processor 64 adjusts the wavelength sweep width of the light source device 12 so that the peak position of the reference point spread function signal becomes the predetermined peak position range (S20). That is, when the wavelength sweep width of the light source device 12 is varied, the peak position of the reference point spread function signal is also varied. Accordingly, the peak position of the reference point spread function signal can come to be within the predetermined position range by adjusting the wavelength sweep width of the light source device 12 according to the shift in the peak position of the reference point spread function signal. Specifically, the processor 64 controls the light source device 12, and adjusts the pulse width of the drive signal (height (voltage) and/or width (time)) outputted from the drive circuit of the light source device 12 to the wavelength sweeping element. Alternatively, the drive cycle of the wavelength sweeping element may be adjusted by adjusting the output cycle of the trigger signal outputted from the light source device 12 to the drive circuit. Due to this, the wavelength sweeping width of the light source device 12 is adjusted, and the peak position of the reference point spread function signal is adjusted to be within the predetermined peak position range. Note that, when the peak position of the reference point spread function signal deviates from the predetermined position range, since the measurement resolution cannot be maintained, accuracy of the position of each part of the eye 100 obtained from the ophthalmic apparatus decreases. In step S20, by adjusting that the peak position of the reference point spread function signal to be within the predetermined peak shape range, the position of each part of the eye 100 can be specified with high accuracy. When step S20 is finished, the processor 64 finishes the process.

As is apparent from the above description, in the ophthalmic apparatus according to the embodiment, the reference point spread function signal is obtained using the light reflected from the first reflection surface 74a of the optical member 74, and the aging of the light source device 12 is determined from the reference point spread function signal. When the wavelength sweeping properties have changed by the aging of the light source device 12, since the adjustment of the wavelength sweeping properties of the above description is performed at an appropriate timing, the measurement sensitivity of the ophthalmic apparatus or the image quality of the captured image can be maintained satisfactorily.

When the aging of the light source device 12 is detected, the adjustment of the data obtainment waiting period and/or the adjustment of the wavelength sweep width need only be performed. Accordingly, the effect of the aging of light source device 12 can be canceled in a short time.

While specific examples of the present teachings have been described above in detail, these examples are merely illustrative and place no limitation on the scope of the patent claims. The technology described in the patent claims also encompasses various changes and modifications to the specific examples described above.

For example, in the above embodiment, the optical calibration system uses reflected light from two reflection surfaces 74a and 74b, but an optical calibration system using reflected light from one reflection surface may be employed. That is, since only one reflection surface 74a is used for adjusting the wavelength sweeping properties of the light source device 12, the optical member 74 is not necessary to have two reflection surfaces, and may be provided with only one reflection surface.

It is explicitly stated that all features disclosed in the description and/or the claims are intended to be disclosed separately and independently from each other for the purpose of original disclosure as well as for the purpose of restricting the claimed invention independent of the composition of the features in the embodiments and/or the claims. It is explicitly stated that all value ranges or indications of groups of entities disclose every possible intermediate value or intermediate entity for the purpose of original disclosure as well as for the purpose of restricting the claimed invention, in particular as limits of value ranges.

## Claims

1. An ophthalmic apparatus that examines an eye by using interference light obtained from reflected light reflected from the eye, the apparatus comprising:
a light source device (12) of a wavelength sweeping type;
an optical reference system (22, 24) configured to guide light from the light source device as reference light;
a calibration member (74) configured to have a predetermined setting length as an optical path length from the light source device, and to include a reflection surface (74a) for reflecting light from the light source device;
a light receiving element (26) configured to receive interference light for calibration produced by both of reflected light reflected at the reflection surface (74a) of the calibration member (74) and the reference light guided by the optical reference system (22, 24); and
a control unit (64),
wherein the light source device (12) includes a light source, a moving part, and a driving part configured to periodically drive the moving part,
the light source device (12) is configured to have the wavelength of the light outputted from the light source device swept periodically, by the light from the light source being outputted via the moving part that is driven periodically,
the light receiving element (26) outputs an interference signal for calibration when the interference light for calibration is received,
the control unit (64) is configured to obtain, according to a driving cycle of the moving part, a reference point spread function signal by obtaining the interference signal for calibration outputted from the light receiving element (26) during a given obtainment period in the driving cycle, **characterized in that**
the control unit (64) is configured to adjust, when a peak shape of the reference point spread function signal deviates from a predetermined shape range, a timing to start the obtainment period in the driving cycle of the moving part so that the peak shape of the reference point spread function signal comes to be within predetermined shape range.

2. The ophthalmic apparatus according to claim 1, wherein
the driving part drives the moving part according to a trigger signal that is periodically outputted,
the control unit (64) obtains the reference point spread function signal by starting the obtainment period when a set time has elapsed since when the trigger signal had been outputted,
and
when the peak shape of the reference point spread function signal deviates from the predetermined shape range, the control unit (64) adjusts the set time from the output of the trigger signal to the start of the obtainment period.

3. The ophthalmic apparatus according to claim 1 or 2, wherein
when a height of the peak shape of the reference point spread function signal becomes lower than a predetermined set height, the control unit (64) adjusts the timing to start the obtainment period in the driving cycle of the moving part so that the height of the peak shape of the reference point spread function signal becomes higher than the predetermined set height.

4. The ophthalmic apparatus according to any one of claims 1 to 3, wherein
when a width of the peak shape of the reference point spread function signal becomes wider than a predetermined set width, the control unit (64) adjusts the timing to start the obtainment period in the driving cycle of the moving part so that the width of the peak shape of the reference point spread function signal becomes narrower than the predetermined set width.

5. The ophthalmic apparatus according to any one of claims 1 to 4, wherein
when a peak position of the reference point spread function signal deviates from a predetermined peak position range, the control unit (64) adjusts a wavelength sweep width of the light source device by controlling the driving part so that the peak position of the reference point spread function signal comes to be within the predetermined peak position range.

6. The ophthalmic apparatus according to claim 5, wherein
the driving part drives the moving part by outputting a pulsed drive signal, and
the control unit (64) adjusts the wavelength sweep width of the light source device by adjusting a pulse width of the drive signal outputted from the driving part.

7. The ophthalmic apparatus according to claim 6, wherein
the control unit (64) adjusts the wavelength sweep width of the light source device (12) by adjusting a cycle of the drive signal outputted from the driving part.

## Patentansprüche

1. Ophthalmologische Vorrichtung, die ein Auge durch Verwenden von Interferenzlicht untersucht, das aus reflektiertem Licht, welches vom Auge reflektiert wurde, erhaltenen wird, mit
einer Lichtquelleneinheit (12) eines Wellenlängendurchlauftyps,
einem optischen Referenzsystem (22, 24), das dazu ausgebildet ist, Licht von der Lichtquelleneinheit als Referenzlicht zu führen,
einem Kalibrationsteil (74), das dazu ausgebildet ist, eine vorbestimmte Einstelllänge als eine optische Pfadlänge von der Lichtquelleneinheit zu haben und eine Reflexionsoberfläche (74a) zum Reflektieren von Licht von der Lichtquelleneinheit aufzuweisen,
einem lichtempfangenden Element (26), das dazu ausgebildet ist, Interferenzlicht zur Kalibration, welches von dem reflektierten Licht, welches an der Reflexionsoberfläche (74a) des Kalibrationsteils (74) reflektiert wird, und dem Referenzlicht, welches von dem optischen Referenzsystem (22, 24) geführt wird, erzeugt wird, zu empfangen, und
einer Steuerungseinheit (64),
wobei die Lichtquelleneinheit (12) eine Lichtquelle, ein sich bewegendes Teil und ein Antriebsteil, welches dazu ausgebildet ist, das sich bewegende Teil periodisch anzutreiben, aufweist,
die Lichtquelleneinheit (12) dazu ausgebildet ist, die Wellenlänge des Lichts, welches von der Lichtquelleneinheit ausgegeben wird, periodisch zu durchlaufen, durch das Licht der Lichtquelle, welches über das sich bewegende Teil, das periodisch angetrieben wird, ausgegeben wird,
das lichtempfangende Element (26) ein Interferenzsignal zur Kalibration ausgibt, wenn das Interferenzlicht zu Kalibration empfangen wird,
die Steuerungseinheit (64) dazu ausgebildet ist, gemäß einem Antriebszyklus des sich bewegenden Teils ein Referenz-Point-Spread-Funktionssignal durch Gewinnen des Interferenzsignals zu Kalibration zu gewinnen, welches von dem lichtempfangenden Element (26) während einer vorgegebenen Gewinnungsperiode in dem Antriebszyklus ausgegeben wird,
**dadurch gekennzeichnet, dass**
die Steuerungseinheit (64) dazu ausgebildet ist, wenn eine Spitzenform des Referenz-Point-Spread-Funktionssignals von einem vorbestimmten Formenbereich abweicht, eine Startzeit der Gewinnungsperiode in dem Antriebszyklus des sich bewegenden Teils derart einzustellen, dass die Spitzenform des Referenz-Point-Spread-Funktionssignals innerhalb des vorbestimmten Formenbereichs kommt.

2. Ophthalmologische Vorrichtung nach Anspruch 1, wobei
das Antriebsteil das sich bewegende Teile gemäß einem Triggersignal, das periodisch ausgegeben wird, antreibt,
die Steuerungseinheit (64) das Referenz-Point-Spread-Funktionssignal durch Starten der Gewinnungsperiode gewinnt, wenn eine festgesetzte Zeit ausgelaufen ist, seitdem das Triggersignal ausgegeben worden war, und,
wenn die Spitzenform des Referenz-Point-Spread-Funktionssignals von dem vorbestimmten Formenbereich abweicht, die Steuerungseinheit (64) die Einstellzeit von der Ausgabe des Triggersignals bis zum Start der Gewinnungsperiode einstellt.

3. Ophthalmologische Vorrichtung nach Anspruch 1 oder 2, wobei,
wenn eine Höhe der Spitzenform des Referenz-Point-Spread-Funktionssignals niedriger wird als eine vorbestimmte festgesetzte Höhe, die Steuerungseinheit (64) die Startzeit der Gewinnungsperiode in dem Antriebszyklus des sich bewegenden Teils derart einstellt, dass die Höhe der Spitzenform des Referenz-Point-Spread-Funktionssignals höher wird als die vorbestimmte festgesetzte Höhe.

4. Ophthalmologische Vorrichtung nach einem der Ansprüche 1 bis 3, wobei,
wenn eine Weite der Spitzenform des Referenz-Point-Spread-Funktionssignals weiter wird als eine vorbestimmte festgesetzte Weite, die Steuerungseinheit (64) die Startzeit der Gewinnungsperiode in dem Antriebszyklus des sich bewegenden Teils derart einstellt, dass die Weite der Spitzenform des Referenz-Point-Spread-Funktionssignals enger wird als die vorbestimmte festgesetzte Weite.

5. Ophthalmologische Vorrichtung nach einem der Ansprüche 1 bis 4, wobei,
wenn eine Spitzenposition des Referenz-Points-Spread-Funktionssignals von einem vorbestimmten Spitzenpositionsbereich abweicht, die Steuerungseinheit (64) eine Wellenlängendurchlaufweite der Lichtquelleneinheit durch Ansteuern des Antriebsteils derart einstellt, dass die Spitzenposition des Referenz-Point-Spread-Funktionssignals innerhalb des vorbestimmten Spitzenpositionsbereichs kommt.

6. Ophthalmologische Vorrichtung nach Anspruch 5, wobei
das Antriebsteil das sich bewegende Teil durch Ausgeben eines gepulsten Antriebssignals antreibt und
die Steuerungseinheit (64) die Wellenlängendurchlaufweite der Lichtquelleneinheit durch Anpassen einer Pulsbreite des Antriebssignals, welches von dem Antriebsteil ausgegeben wird, einstellt.

7. Ophthalmologische Vorrichtung nach Anspruch 6, wobei
die Steuerungseinheit (64) die Wellenlängendurchlaufweite der Lichtquelleneinheit (12) durch Einstellen eines Zyklus des Antriebssignals, welches von dem Antriebsteil ausgegeben wird, einstellt.

## Revendications

1. Appareil ophtalmique qui examine un oeil en utilisant une lumière d'interférence obtenue à partir d'une lumière réfléchie qui est réfléchie depuis l'oeil, l'appareil comprenant :
un dispositif de source de lumière (12) d'un type à balayage de longueur d'onde ;
un système de référence optique (22, 24) configuré pour guider de la lumière provenant du dispositif de source de lumière en tant que lumière de référence ;
un élément d'étalonnage (74) configuré pour avoir une longueur de réglage prédéterminée en tant que longueur de chemin optique depuis le dispositif de source de lumière, et pour inclure une surface de réflexion (74a) pour réfléchir de la lumière provenant du dispositif de source de lumière ;
un élément de réception de lumière (26) configuré pour recevoir de la lumière d'interférence pour étalonnage produite à la fois par la lumière réfléchie qui est réfléchie au niveau de la surface de réflexion (74a) de l'élément d'étalonnage et la lumière de référence guidée par le système de référence optique (22, 24) ;
et
une unité de commande (64),
dans lequel le dispositif de source de lumière (12) inclut une source de lumière, une partie mobile et une partie d'entraînement configurée pour entraîner périodiquement la partie mobile,
le dispositif de source de lumière (12) est configuré pour avoir la longueur d'onde de la lumière sortie depuis le dispositif de source de lumière balayée périodiquement, par la lumière provenant de la source de lumière sortie via la partie mobile qui est entraînée périodiquement,
l'élément de réception de lumière (26) sort un signal d'interférence pour étalonnage quand la lumière d'interférence pour étalonnage est reçue,
l'unité de commande (64) est configurée pour obtenir, en fonction d'un cycle d'entraînement de la partie mobile, un signal de fonction de propagation de point de référence en obtenant le signal d'interférence pour étalonnage sorti depuis l'élément de réception de lumière (26) durant une période d'obtention donnée dans le cycle d'entraînement, **caractérisé en ce que**
l'unité de commande (64) est configurée pour ajuster, quand une forme de crête du signal de fonction de propagation de point de référence dévie d'une plage de forme prédéterminée, un moment pour démarrer la période d'obtention dans le cycle d'entraînement de la partie mobile de telle sorte que la forme de crête du signal de fonction de propagation de point de référence parvient à se trouver dans une plage - de forme prédéterminée.

2. Appareil ophtalmique selon la revendication 1, dans lequel
la partie d'entraînement entraîne la partie mobile en fonction d'un signal de déclenchement qui est périodiquement sorti,
l'unité de commande (64) obtient le signal de fonction de propagation de point de référence en démarrant la période d'obtention quand un temps de consigne s'est écoulé depuis que le signal de déclenchement a été sorti, et
quand la forme de crête du signal de fonction de propagation de point de référence dévie de la plage de forme prédéterminée, l'unité de commande (64) ajuste le temps de consigne depuis la sortie du signal de déclenchement jusqu'au démarrage de la période d'obtention.

3. Appareil ophtalmique selon la revendication 1 ou 2, dans lequel
quand une hauteur de la forme de crête du signal de fonction de propagation de point de référence devient inférieure à une hauteur de consigne prédéterminée, l'unité de commande (64) ajuste le moment où démarrer la période d'obtention dans le cycle d'entraînement de la partie mobile de telle sorte que la hauteur de la forme de crête du signal de fonction de propagation de point de référence devient supérieure à la hauteur de consigne prédéterminée.

4. Appareil ophtalmique selon l'une quelconque des revendications 1 à 3, dans lequel
quand une largeur de la forme de crête du signal de fonction de propagation de point de référence devient plus large qu'une largeur de consigne prédéterminée, l'unité de commande (64) ajuste le moment où démarrer la période d'obtention dans le cycle d'entraînement de la partie mobile de telle sorte que la largeur de la forme de crête du signal de fonction de propagation de point de référence devient plus étroite que la largeur de consigne prédéterminée.

5. Appareil ophtalmique selon l'une quelconque des revendications 1 à 4, dans lequel
quand une position de crête du signal de fonction de propagation de point de référence dévie d'une plage de position de crête prédéterminée, l'unité de commande (64) ajuste une largeur de balayage de longueur d'onde du dispositif de source de lumière en commandant la partie d'entraînement de telle sorte que la position de crête du signal de fonction de propagation de point de référence parvient à se trouver dans la plage de position de crête prédéterminée.

6. Appareil ophtalmique selon la revendication 5, dans lequel
la partie d'entraînement entraîne la partie mobile en sortant un signal d'entraînement à impulsions, et l'unité de commande (64) ajuste la largeur de balayage de longueur d'onde du dispositif de source de lumière en ajustant une largeur d'impulsion du signal d'entraînement sorti depuis la partie d'entraînement.

7. Appareil ophtalmique selon la revendication 6, dans lequel
l'unité de commande (64) ajuste la largeur de balayage de longueur d'onde du dispositif de source de lumière (12) en ajustant un cycle du signal d'entraînement sorti depuis la partie d'entraînement.9
